# EUROPÄISCHE PATENTANMELDUNG

(11) **EP 0 771 561 A2**
(43) Veröffentlichungstag der Anmeldung: **07.05.1997**
(21) Anmeldenummer: 96116976.0
(22) Anmeldetag: 23.10.1996
(51) Int. Cl.: A61K 31/045, A61K 31/085

(54) **Antimycotische, insbesondere gegen Kopfschuppen wirksame, Zubereitungen mit einem wirksamen Gehalt an aromatischen Alkoholen**

(30) Priorität: 30.10.1995 DE 19540464
(71) Anmelder: Beiersdorf Aktiengesellschaft, D-20245 Hamburg (DE)
(72) Erfinder: Wolf, Florian, Dr., 20251 Hamburg (DE); Klier, Manfred, Dr., 21521 Aumühle (DE); Schmidt-Lewerkühne, Hartmut, Dr., 22869 Schenefeld (DE)

(57) **Zusammenfassung**

Verwendung einer oder mehreren Arylverbindungen der allgemeinen Strukturformel 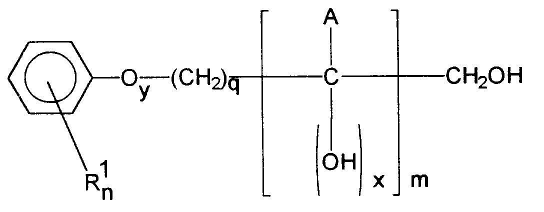 wobei R¹ darstellen kann: H, CH₃, OCH₃, NH₂, und wobei bis zu fünf gleiche oder verschiedene Reste R¹ bzw. beliebige Kombinationen gleicher und verschiedener solcher Reste innerhalb eines Moleküls auftreten können, entsprechend n = 1 - 5,
wobei A darstellen kann: H, Methyl, Ethyl
wobei der Index q Werte von 0 - 10 annehmen kann
wobei der Index m Werte von 0 - 10 annehmen kann,
wobei der Index y die Werte 0 oder 1 annehmen kann,
wobei der Index x die Werte 0 oder 1 annehmen kann, und wobei innerhalb eines Moleküls, wenn m > 1, für einzelne Kohlenstoffatome x identische, aber auch unterschiedliche Werte annehmen kann, und
wobei gewährleistet sein muß, daß die Arylverbindung bzw. -verbindungen einzeln oder in Kombination kosmetisch bzw. pharmazeutisch akzeptabel ist bzw. sind,
als antimycotisches Wirkprinzip in kosmetischen oder dermatologischen Zubereitungen.

## Beschreibung

Die vorliegende Erfindung betrifft antimycotisch wirksame und gegen den Befall mit Mycobionten geschützte Zubereitungen, bevorzugt kosmetischen oder dermatologischen Zubereitungen.

Pilze, auch Fungi [fungus = lat. Pilz], Mycota [µυκησ = grch. Pilz] oder Mycobionten genannt, zählen zu den Eucaryonten. Eucaryonten sind Lebewesen, deren Zellen (Eucyten) im Gegensatz zu denen der sogenannten Procaryonten (Procyten) über einen durch Kernhülle und Kemmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaffgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in Haut, Haare, Haarfollikel sowie Finger- oder Zehennägel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityrosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm und nur sehr schlecht zu behandeln sind weiterhin Pilzerkrankungen der Finger- und Fußnägelbereiche (Onychomycosen).

Ferner sind Superinfektionen der Haut durch Pilze nicht selten.

Die gesunde menschliche Haut ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog, extreme UV-Lichtexposition), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen. Meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird. Darüberhinaus sind viele der zu bekämpfenden Mikroorganismen im Laufe der Zeit durch bisweilen leichtfertige und voreilige Therapien mit Antibiotika deutlich resistenter gegenüber diesen geworden, so daß die topische Verwendung von Antibiotika zur Behandlung der geschilderten Erscheinungen zusehends obsolet wird.

Eine Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nenneswerte Einbußen erleidet.

Darüberhinaus war es eine Aufgabe der vorliegenden Erfindung, Substanzen und kosmetische bzw. dermatologische Zubereitungen, solche Substanzen enthaltend, zur Verfügung zu stellen, die prophylaktisch gegen Superinfektionen wirken.

Schließlich können durch Mycobiontenbefall Zubereitungen, insbesondere Nahrungsmittel, aber auch Kosmetika und dergleichen zerstört werden. Der Schutz vor Infektion erstreckt sich daher auch auf die Produkte selbst.

Behandelt werden Dermatomycosen medikamentös oder mit anderen Methoden, beispielsweise mit Lichtbestrahlung. Gängige Medikamente enthalten Salicylsäure, Kresol, 1-Menthol und andere, die alle äußerlich angewandt werden und regelmäßig gute Heilerfolge zeitigen.

Dennoch haben die bisher bekannten antimycotisch wirksamen Mittel den Nachteil daß sie unangenehm riechen und/oder die beeinträchtigte Hautpartie zusätzlich reizen. In schweren Fällen von Dermatomycosen kann durch das Medikament sogar Schmerz ausgelöst werden.

Eine weitere Aufgabe der vorliegenden Erfindung war also, antimycotisch wirksame Mittel zur Verfügung zu stellen, welche die Nachteile des Standes der Technik nicht aufweisen. Insbesondere sollten Wirkstoffe zugängig gemacht werden, die folgende Bedingungen erfüllen:
1) Die biologischen Vorgänge der Haut dürfen nicht beeinträchtigt werden.
2) Die Wirkstoffe sollen keinen ausgeprägten Eigengeruch besitzen.
3) Sie sollen bei Überdosierung oder sonstiger nicht bestimmungsgemäßer Anwendung unschädlich sein.
4) Sie sollen sich nach wiederholter Anwendung nicht auf der Haut anreichern.
5) Pathogene und fakultativ pathogene Mycobionten sollen wirksam bekämpft, die physiologische Mikroflora der Haut im übrigen aber geschont werden.
6) Die wirksamen Prinzipien sollen sich gut in übliche kosmetische oder dermatologische Formulierungen einarbeiten lassen.
7) Die Wirkstoffe sollen die Haut nicht reizen.
8) Zubereitungen, diese Wirkstoffe enthaltend, sollen ebenfalls vor Befall mit Mycobionten geschützt sein.

Es wurde gefunden, und darin liegt die Lösung all dieser Aufgaben, daß die Verwendung einer oder mehreren Arylverbindungen der allgemeinen Strukturformel 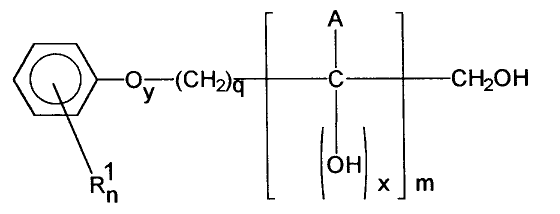 wobei R¹ darstellen kann: H, CH₃, OCH₃, NH₂, und wobei bis zu fünf gleiche oder verschiedene Reste R¹ bzw. beliebige Kombinationen gleicher und verschiedener solcher Reste innerhalb eines Moleküls auftreten können, entsprechend n = 1 - 5,
wobei A darstellen kann: H, Methyl, Ethyl
wobei der Index q Werte von 0 - 10 annehmen kann
wobei der Index m Werte von 0 - 10 annehmen kann,
wobei der Index y die Werte 0 oder 1 annehmen kann,
wobei der Index x die Werte 0 oder 1 annehmen kann, und wobei innerhalb eines Moleküls, wenn m > 1, für einzelne Kohlenstoffatome x identische, aber auch unterschiedliche Werte annehmen kann, und
wobei gewährleistet sein muß, daß die Arylverbindung bzw. -verbindungen einzeln oder in Kombination kosmetisch bzw. pharmazeutisch akzeptabel ist bzw. sind,
als antimycotisches Wirkprinzip in kosmetischen oder dermatologischen Zubereitungen, den Nachteilen des Standes der Technik abhilft.

Es ist zwar aus der DE-OS 37 40 186 bekannt, Phenoxyethanol als antimikrobiell wirksamen Bestandteil kosmetischer Desodorantien einzusetzen. Dieser Stand der Technik betrifft jedoch synergistische Wirkstoffkombinationen mit anderen Substanzen, welche nicht Gegenstand der vorliegenden Erfindung sind und ihre Verwendung zur Bekämpfung den menschlichen Schweiß zersetzender Bakterien, nicht Mycota. Er konnte daherkeinen Hinweis auf die erfindungsgemäße Verwendung geben.

Erfindungsgemäß bevorzugte Arylverbindung werden gewählt aus der Gruppe Phenoxyethanol, Anisalkohol, 2-Methyl-5-phenyl-pentan-1-ol.

Phenoxyethanol ist gekennzeichnet durch die Strukturformel 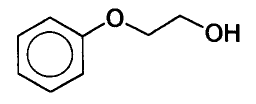 Anisalkohol ist gekennzeichnet durch die Strukturformel 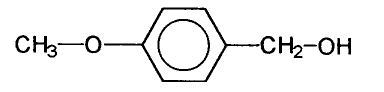 2-Methyl-5-phenyl-pentan-1-ol ist gekennzeichnet durch die Strukturformel

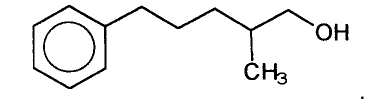

Die erfindungsgemäß wirksamen Arylverbindungen sind ausgezeichnet wirksam gegen Mycobionten, insbesondere in deren Auswirkungsform der Dermatomycosen. Dabei sind die erfindungsgemäßen Wirkstoffkombinationen insbesondere befähigt, das Wachstum von Hefen, insbesondere der Pityrosporum-Arten, namentlich Pityrosporum ovale, zu verhindern.

Es hat sich ferner in überraschender Weise herausgestellt, daß die erfindungsgemäß wirksamen Arylverbindungen die Bildung von seborrhoischen Erscheinungen, insbesondere Kopfschuppen, verhindern sowie bereits vorhandene seborrhoische Erscheinungen, insbesondere Kopfschuppen, zu beseitigen.

Schließlich hat sich herausgestellt, daß die erfindungsgemäß wirksamen Arylverbindungen den Verderb organischer Substanz, insbesondere kosmetischer und dermatologischer Zubereitungen, durch den Befall mit Mycobionten verhindern können, wenn sie diesen Zubereitungen zugesetzt werden.

Erfindungsgemäß sind somit auch ein Verfahren zur Bekämpfung von Mycobionten, dadurch gekennzeichnet, daß die erfindungsgemäß wirksamen Arylverbiundungen, gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, daß diesen organischen Produkten erfindungsgemäß wirksame Arylverbindungen in wirksamer Menge zugegeben werden.

Ferner war erstaunlich, daß die erfindungsgemäß wirksamen Arylverbindungen besonders gut wirksam sind gegen den für das Entstehen von Kopfschuppen verantwortlichen Keim Pityrosporum ovale und verwandte Keime. Eine bevorzugte Ausführungsform der vorliegenden Erfindung sind mithin gegen Kopfschuppen anzuwendende Formulierungen, beispielsweise Antischuppenshampoos.

Die erfindungsgemäß wirksamen Zubereitungen sind besonders vorteilhaft dadurch gekennzeichnet, daß die Arylverbindung oder die Arylverbindungen in Konzentrationen von 0,001 - 10,00 Gew.-%, bevorzugt 0,05 - 5,00 Gew.-%, besonders bevorzugt 0,01 - 3,00 Gew.-%, jeweils bezogen auf das Gesamtgewicht der Zusammensetzung, vorliegt oder vorliegen.

Erfindungsgemäß werden die Arylverbindungen bevorzugt in kosmetischen oder dermatologischen Zusammensetzungen eingesetzt in einem Gehalt von 0,005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-% an den erfindungsgemäßen Arylverbindungen, ganz besonders vorteilhaft 0,5 - 3,0 Gew.-%, jeweils bezogen auf das Gesamtgewicht der Zusammensetzung.

Die erfindungsgemäß wirksamen Arylverbindungen lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z.B. Nagellacke, Nagellackentferner, Nagelbalsame) und dergleichen.

Ganz besonders vorteilhaft liegen die erfindungsgemäß wirksamen Arylverbindungen in Form von Antischuppen-Shampoos vor.

Es ist auch möglich und gegebenenfalls vorteilhaft, die erfindungsgemäß wirksamen Arylverbindungen mit anderen Wirkstoffen zu kombinieren, beispielsweise mit antimikrobiell wirksamen Stoffen.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,0 zu wählen.

Die erfindungsgemäßen kosmetischen oder dermatologischen Zubereitungen können wie üblich zusammengesetzt sein und zur Behandlung der Haut oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugt können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Kosmetische Zubereitungen gemäß der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise herkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide, Polyglycolether-Derivate.

Kosmetische Zubereitungen, die kosmetische Reinigungszubereitungen für die Haut darstellen, können in flüssiger oder fester Form vorliegen. Sie enthalten vorzugsweise mindestens eine anionische, nicht-ionische oder amphotere oberflächenaktive Substanz oder Gemische daraus, gegebenenfalls Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Kosmetische Zubereitungen, die ein Shampoonierungsmittel oder eine Wasch-, Dusch- oder Badezubereitung darstellen, enthalten vorzugsweise mindestens eine anionische, nicht-ionische oder amphotere oberflächenaktive Substanz oder Gemische daraus, eine erfindungsgemäß wirksame Arylverbindung im wäßrigen Medium und Hilfsmittel, wie sie üblicherweise dafür verwendet werden. Die oberflächenaktive Substanz kann in einer Konzentration zwischen 1 Gew.-% und 50 Gew.-% in dem Shampoonierungsmittel, bzw. der Wasch-, Dusch- oder Badezubereitung, vorliegen.

Liegt die kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische davon sowie mindestens eine erfindungsgemäß wirksame Arylverbindung.

Kosmetische und dermatologische Zubereitungen zur Behandlung und Pflege der Haare, die die erfindungsgemäß wirksame Arylverbindungen enthalten, können als Emulsionen vorliegen, die vom nicht-ionischen oder anionischen Typ sind. Nicht-ionische Emulsionen enthalten neben Wasser Öle oder Fettalkohole, die beispielsweise polyethoxyliert oder polypropoxyliert sein können, oder auch Gemische aus den beiden organischen Komponenten. Diese Emulsionen enthalten gegebenenfalls kationische oberflächenaktive Substanzen. Anionische Emulsionen sind vorzugsweise vom Typ einer Seife und enthalten mindestens eine erfindungsgemäße ethoxylierte oder propoxylierte organische Verbindung mit anionischem oder nicht-ionischem Charakter.

Kosmetische und dermatologische Zubereitungen zur Behandlung und Pflege der Haare können als Gele vorliegen, die neben den erfindungsgemäß wirksamen Arylverbindungen und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglykolstearat oder -distearat, enthalten. Das Verdickungsmittel ist im Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäß wirksamen Arylverbindungen in einem für die Haare bestimmten Mittel 0,01 Gew.-% bis 10 Gew.%, insbesondere 0,5 Gew.-% bis 5 Gew.-%, bezogen auf das Gesamtgewicht der Zubereitungen.

Die folgenden Beispiele sollen die Verkörperungen der vorliegenden Erfindungen verdeutlichen. Die Angaben beziehen sich stets auf Gewichts-%, sofern nicht andere Angaben gemacht werden.

### Beispiel 1

| W/O-Crème | | | |
|---|---|---|---|
| | I | II | III |
| Paraffinöl | 10,00 | 10,00 | 10,00 |
| Ozokerit | 4,00 | 4,00 | 4,00 |
| Vaseline | 4,00 | 4,00 | 4,00 |
| pflanzliches Öl | 10,00 | 10,00 | 10,00 |
| Wollwachsalkohol | 2,00 | 2,00 | 2,00 |
| Aluminiumstearat | 0,40 | 0,40 | 0,40 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 2

| W/O-Lotion | | | |
|---|---|---|---|
| | I | II | III |
| Paraffinöl | 25,00 | 25,00 | 25,00 |
| Siliconöl | 2,00 | 2,00 | 2,00 |
| Ceresin | 1,50 | 1,50 | 1,50 |
| Wollwachsalkohol | 0,50 | 0,50 | 0,50 |
| Glucosesesquiisostearat | 2,50 | 2,50 | 2,50 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 3

| O/W-Lotion | | | |
|---|---|---|---|
| | I | II | III |
| Paraffinöl | 5,00 | 5,00 | 5,00 |
| Isopropylpalmitat | 5,00 | 5,00 | 5,00 |
| Cetylalkohol | 2,00 | 2,00 | 2,00 |
| Bienenwachs | 2,00 | 2,00 | 2,00 |
| Ceteareth-20 | 2,00 | 2,00 | 2,00 |
| PEG-20-Glycerylstearat | 1,50 | 1,50 | 1,50 |
| Glycerin | 3,00 | 3,00 | 3,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 4

| O/W-Crème | | | |
|---|---|---|---|
| | I | II | III |
| Pflanzliches Öl | 10,00 | 10,00 | 10,00 |
| Cetylalkohol | 2,00 | 2,00 | 2,00 |
| Glycerinmonostearat | 1,50 | 1,50 | 1,50 |
| PEG-30-Glycerylstearat | 2,00 | 2,00 | 2,00 |
| Glycerin | 3,00 | 3,00 | 3,00 |
| Isopropylpalmitat | 5,00 | 5,00 | 5,00 |
| Carbopol 980 (neutralisiert) | 0,30 | 0,30 | 0,30 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 5

| Salbe | | | |
|---|---|---|---|
| | I | II | III |
| Vaseline | 36,00 | 36,00 | 36,00 |
| Ceresin | 10,00 | 10,00 | 10,00 |
| Zinkoxid | 4,00 | 4,00 | 4,00 |
| Pflanzliches Öl | 20,00 | 20,00 | 20,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Paraffinöl | .............. ad 100,00 .............. | | |

### Beispiel 6

| Hautöl | | | |
|---|---|---|---|
| | I | II | III |
| Cetylpalmitat | 3,00 | 3,00 | 3,00 |
| C_{12 - 15}- Alkylbenzoat | 2,00 | 2,00 | 2,00 |
| Polyisobuten | 10,00 | 10,00 | 10,00 |
| Squalan | 2,00 | 2,00 | 2,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Paraffinöl | .............. ad 100,00 .............. | | |

### Beispiel 7

| Badeöl | | | |
|---|---|---|---|
| | I | II | III |
| Paraffinöl | 20,00 | 20,00 | 20,00 |
| PEG-40-hydriertes Rizinusöl | 5,00 | 5,00 | 5,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Sojaöl | .............. ad 100,00 .............. | | |

### Beispiel 8

| Lippenstift | | | |
|---|---|---|---|
| | I | II | III |
| Ceresin | 8,00 | 8,00 | 8,00 |
| Bienenwachs | 4,00 | 4,00 | 4,00 |
| Carnaubawachs | 2,00 | 2,00 | 2,00 |
| Vaseline | 40,00 | 40,00 | 40,00 |
| Hydriertes Rizinusöl | 4,00 | 4,00 | 4,00 |
| Caprylic/Capric Triglyceride | 6,00 | 6,00 | 6,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Paraffinöl | .............. ad 100,00 .............. | | |

### Beispiel 9

| Pflegemaske | | | |
|---|---|---|---|
| | I | II | III |
| PEG-50 Lanolin | 0,50 | 0,50 | 0,50 |
| Glycerylstearat | 2,00 | 2,00 | 2,00 |
| Sonnenblumenkernöl | 3,00 | 3,00 | 3,00 |
| Bentonit | 8,00 | 8,00 | 8,00 |
| Kaolin | 35,00 | 35,00 | 35,00 |
| Zinkoxid | 5,00 | 5,00 | 5,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 10

| Liposomenhaltiges Gel | | | |
|---|---|---|---|
| | I | II | III |
| Lecithin | 6,00 | 6,00 | 6,00 |
| Pflanzliches Öl | 12,50 | 12,50 | 12,50 |
| Hydrolysiertes Kollagen | 2,00 | 2,00 | 2,00 |
| Xanthan Gum | 1,40 | 1,40 | 1,40 |
| Butylenglycol | 3,00 | 3,00 | 3,00 |
| Phenoxyethanol | 0,50 | - | - |
| Anisalkohol | - | 0,50 | - |
| 2-Methyl-5-phenyl-pentan-1-o | - | - | 0,50 |
| Parfum, Konservierungsstoffe | ................... q.s. ................... | | |
| Wasser, VES | .............. ad 100,00 .............. | | |

### Beispiel 11

| Duschpräparat mit Rückfetter | | | |
|---|---|---|---|
| | I | II | II |
| Cocoamidodiacetat | 10,00 | 10,00 | 10,00 |
| Natriumlaurylsulfat | 25,00 | 25,00 | 25,00 |
| Kalium Cocyl Hydrolysiertes Kollagen | 5,00 | 5,00 | 5,00 |
| Macadamianußöl | 5,00 | 5,00 | 5,00 |
| Natriumchlorid | 0,60 | 0,60 | 0,60 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 12

| Seifenstück | | | |
|---|---|---|---|
| | I | II | III |
| Na-Salz aus Talgfettsäuren | 60,00 | 60,00 | 60,00 |
| Na-Salz aus Kokosöl | 28,00 | 28,00 | 28,00 |
| Natriumchlorid | 0,50 | 0,50 | 0,50 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 13

| Syndetseife | | | |
|---|---|---|---|
| | I | II | III |
| Natriumlaurylsulfat | 30,00 | 30,00 | 30,00 |
| Natriumsulfosuccinat | 10,00 | 10,00 | 10,00 |
| Kaliumcocoyl hydrolysiertes Kollagen | 2,00 | 2,00 | 2,00 |
| Dimethicon Copolyol | 2,00 | 2,00 | 2,00 |
| Paraffin | 2,00 | 2,00 | 2,00 |
| Maisstärke | 10,00 | 10,00 | 10,00 |
| Talcum | 10,00 | 10,00 | 10,00 |
| Glycerin | 3,00 | 3,00 | 3,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 14

| Haarpflegemittel | | | |
|---|---|---|---|
| | I | II | III |
| TEA-Cocoyl hydrolysiertes Kollagen | 30,00 | 30,00 | 30,00 |
| Monoethanolaminlaurylsulfat | 25,00 | 25,00 | 25,00 |
| Mandelöl | 2,00 | 2,00 | 2,00 |
| Natriumchlorid | 1,00 | 1,00 | 1,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 15

| Pflegeshampoo | | | |
|---|---|---|---|
| | I | II | III |
| Natriumlaurylsulfat | 34,00 | 34,00 | 34,00 |
| Dinatriumlaurylsulfosuccinat | 6,00 | 6,00 | 6,00 |
| Cocoamidopropylbetain | 10,00 | 10,00 | 10,00 |
| Glycoldistearat | 5,00 | 5,00 | 5,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 16

| Haarkur | | | |
|---|---|---|---|
| | I | II | III |
| Cetylalkohol | 5,00 | 5,00 | 5,00 |
| Caprylic/Capric Triglyceride | 3,00 | 3,00 | 3,00 |
| Petrolatum | 2,00 | 2,00 | 2,00 |
| Wollwachsalkohol | 0,50 | 0,50 | 0,50 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 17

| Haarspülung | | | |
|---|---|---|---|
| | I | II | III |
| Cocoamidopropylbetain | 5,00 | 5,00 | 5,00 |
| Cetylalkohol | 2,00 | 2,00 | 2,00 |
| Propylenglycol | 2,00 | 2,00 | 2,00 |
| Citronensäure | 0,30 | 0,30 | 0,30 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 18

| Haarfestiger | | | |
|---|---|---|---|
| | I | II | III |
| Polyvinylpyrrolidon/Vinylacetat/ Vinylpropionat-Copolymer | 5,00 | 5,00 | 5,00 |
| Ethanol | 45,00 | 45,00 | 45,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 19

| Frisiercrème | | | |
|---|---|---|---|
| | I | II | III |
| Vaseline | 4,00 | 4,00 | 4,00 |
| Cetearylalkohol | 4,00 | 4,00 | 4,00 |
| PEG-40-hydriertes Rizinusöl | 2,00 | 2,00 | 2,00 |
| Isopropylpalmitat | 5,00 | 5,00 | 5,00 |
| Citronensäure | 1,00 | 1,00 | 1,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 20

| Rasierschaum | | | |
|---|---|---|---|
| | I | II | III |
| Stearinsäure | 7,00 | 7,00 | 7,00 |
| Natriumlaurylsulfat | 3,00 | 3,00 | 3,00 |
| Stearylalkohol | 1,00 | 3,00 | 3,00 |
| Glycerin | 5,00 | 5,00 | 5,00 |
| Triethanolamin | 3,60 | 3,60 | 3,60 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 21

| Fußcrème | | | |
|---|---|---|---|
| | I | II | III |
| Soluan 5 | 2,00 | 2,00 | 2,00 |
| Methylsalicylat | 5,00 | 5,00 | 5,00 |
| Caprylic/Capric Triglyceride | 10,00 | 10,00 | 10,00 |
| Stearinsäure | 5,00 | 5,00 | 5,00 |
| Cetylalkohol | 1,00 | 1,00 | 1,00 |
| Glycerin | 2,00 | 2,00 | 2,00 |
| Dimethicon | 1,00 | 1,00 | 1,00 |
| Carbopol 984 | 0,50 | 0,50 | 0,50 |
| Triethanolamin | 1,50 | 1,50 | 1,50 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 22

| Aerosolspray | | | |
|---|---|---|---|
| | I | II | III |
| Octyldodecanol | 0,50 | 0,50 | 0,50 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Ethanol | .............. ad 100,00 .............. | | |

Die durch Zusammennmischung der jeweiligen Bestandteile erhaltene flüssige Phase wird zusammen mit einem Propan-Butan-Gemisch (2:7) im Verhältnis 39:61 in Aerosolbehälter abgefüllt.

### Beispiel 23

| Pumpspray | | | |
|---|---|---|---|
| | I | II | III |
| PEG-40-Hydriertes Rizinusöl | 2,00 | 2,00 | 2,00 |
| Glycerin | 1,00 | 1,00 | 1,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 24

| Roll-on-Gel | | | |
|---|---|---|---|
| | I | II | III |
| 1,3-Butylenglycol | 2,00 | 2,00 | 2,00 |
| PEG-40-Hydriertes Rizinusöl | 2,00 | 2,00 | 2,00 |
| Hydroxyethylcellulose | 0,50 | 0,50 | 0,50 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 25

| Roll-on-Emulsion | | | |
|---|---|---|---|
| | I | II | III |
| Tricetearethphosphat | 0,30 | 0,30 | 0,30 |
| Octyldodecanol | 2,00 | 2,00 | 2,00 |
| C_{12 - 15}-Alkylbenzoat | 2,00 | 2,00 | 2,00 |
| C_{10 - 30}-Alkylacrylat | 0,15 | 0,15 | 0,15 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Wasser, VES | .............. ad 100,00 .............. | | |

### Beispiel 26

| Wachsstift | | | |
|---|---|---|---|
| | I | II | III |
| Hydriertes Rizinusöl | 5,00 | 5,00 | 5,00 |
| Bienenwachs | 6,00 | 6,00 | 6,00 |
| Ceresin | 30,00 | 30,00 | 30,00 |
| C_{12 - 15}-Alkylbenzoat | 17,00 | 17,00 | 17,00 |
| Phenoxyethanol | 0,50 | - | - |
| Anisalkohol | - | 0,50 | - |
| 2-Methyl-5-phenyl-pentan-1-ol | - | - | 0,50 |
| Parfum, Konservierungsstoffe | ................... q.s. ................... | | |
| Octyldodecanol | .............. ad 100,00 .............. | | |

## Patentansprüche

1. Verwendung einer oder mehreren Arylverbindungen der allgemeinen Strukturformel 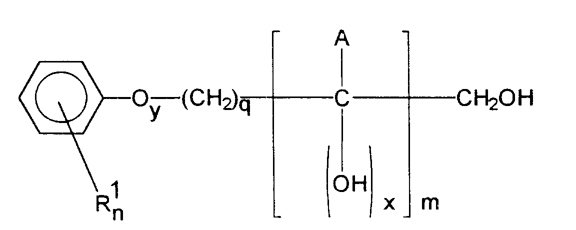 wobei R¹ darstellen kann: H, CH₃, OCH₃, NH₂, und wobei bis zu fünf gleiche oder verschiedene Reste R¹ bzw. beliebige Kombinationen gleicher und verschiedener solcher Reste innerhalb eines Moleküls auftreten können, entsprechend n = 1 - 5,
wobei A darstellen kann: H, Methyl, Ethyl
wobei der Index q Werte von 0 - 10 annehmen kann
wobei der Index m Werte von 0 - 10 annehmen kann,
wobei der Index y die Werte 0 oder 1 annehmen kann,
wobei der Index x die Werte 0 oder 1 annehmen kann, und wobei innerhalb eines Moleküls, wenn m > 1, für einzelne Kohlenstoffatome x identische, aber auch unterschiedliche Werte annehmen kann, und
wobei gewährleistet sein muß, daß die Arylverbindung bzw. -verbindungen einzeln oder in Kombination kosmetisch bzw. pharmazeutisch akzeptabel ist bzw. sind,
als antimycotisches Wirkprinzip in kosmetischen oder dermatologischen Zubereitungen.

2. Verwendung nach Anspruch 1, dadurch gekennzeichnet, daß die Arylverbindung gewählt wird aus der Gruppe Phenoxyethanol, Anisalkohol, 2-Methyl-5-phenylpentan-1-ol.

3. Verwendung nach Anspruch 1, dadurch gekennzeichnet, daß in kosmetischen Zubereitungen 0,005 - 50,0 Gew.-% an Arylverbindungen, insbesondere 0,01 - 20,0 Gew.-%, bezogen auf das Gesamtgewicht der Zusammensetzung bevorzugt sind.
